# EUROPEAN PATENT SPECIFICATION

(11) **EP 4 192 440 B1**
(45) Date of publication and mention of the grant of the patent: **04.09.2024**
(21) Application number: 21749790.8
(22) Date of filing: 22.07.2021
(51) Int. Cl.: A61K 9/70, A61K 47/06, A61K 47/10, A61K 47/12, A61K 47/14, A61K 47/18, A61K 47/22, A61K 47/32, A61K 47/34, A61K 47/38, A61K 31/135, A61P 23/02, A61P 25/24, A61P 29/02

(54) **ESKETAMINE-SUSPENSION-TTS**
ESKETAMIN-SUSPENSIONS-TTS
TTS COMPRENANT UNE SUSPENSION D'ESKÉTAMINE

(30) Priority: 06.08.2020 EP 20189832
(43) Date of publication of application: 14.06.2023
(73) Proprietor: LTS Lohmann Therapie-Systeme AG, 56626 Andernach (DE)
(72) Inventor: HAMMES, Florian, 56626 Andernach (DE); TOMELERI, Anja, 56567 Neuwied (DE); KLEUDGEN, Tobias, 56729 Ettringen (DE)
(74) Representative: Meissner Bolte Partnerschaft mbB
(86) International application number: PCT/EP2021/070578
(87) International publication number: WO 2022/028913

(56) References cited:
- EP-A1- 1 386 604
- WO-A1-2017/003935
- WO-A2-03/015699
- US-A1- 2018 064 655
- HEALTHCARE SOLUTIONS: "Dow Corning BIO-PSA Amine-Compatible Silicone Adhesives", 9 August 2016 (2016-08-09), XP055765838, Retrieved from the Internet <URL:http://www.healthcare-plus.com.tw/big5/pdf/02-02.pdf> [retrieved on 20210115]

## Description

The present invention relates to a transdermal therapeutic system (TTS) comprising ketamine as active ingredient. The invention further concerns such a system for use as drug, in particular for use in the treatment of depression and/or pain.

In the past years, transdermal therapeutic systems have become increasingly important as dosage form for treating numerous diseases, because they have advantages over common dosage forms. Those are, for example, a precise and constant drug release, which is necessary for a constant concentration of the active ingredient in the blood plasma. Further, the first pass effect can be avoided and compliance can be increased, because the patient does not need to take tablets regularly. An advantage of transdermal therapeutic systems over other topical application systems such as ointments or creams is that they can be applied area accurate and therefore dosage accurate and that there is no risk of incidental wiping off the ointment with contamination of other regions. Further, ointments or tablets must be administered regularly, because a sustained release of the active ingredient usually cannot be achieved otherwise.

US 2018/064655 discloses a transdermal patch comprising (2R,6R)-hydroxynorketamine or a conjugate thereof.

EP1386604A1 discloses an improved Transdermal Delivery System (TDS) comprising a backing layer inert to the components of the matrix, a self-adhesive matrix containing an amine-functional drug and a protective foil or sheet to be removed prior to use, characterized in that the self-adhesive matrix consists of a solid or semi-solid semi-permeable polymer.

Via the URL: http://www.healthcare-plus.com.tw/big5/pdf/02-02.pdf Amine-Compatible Silicone Adhesives were found on 2016-08-09.

A few years ago, it was believed that the implementation of active ingredients in transdermal therapeutic system would be easily achievable, so that this application form would be available for a large number of active ingredients. However, it turned out that this is not correct, because the molecular transport of ingredients via the skin poses a limiting factor. Thus, intense research is always required in order to provide transdermal therapeutic systems for the administration of new active ingredients.

The active ingredient ketamine is long known for the treatment of pain. Recently, it has also been discovered that ketamine is suitable for the treatment of psychological disorders, in particular of depression.

A transdermal therapeutic system provides an attractive option for the administration of ketamine.

Transdermal therapeutic systems for the administration of ketamine are known from the prior art.

For example, WO 2017/003935 A1 and WO 2018/195318 A1 disclose a TTS for the administration of ketamine, wherein a pressure sensitive adhesive is employed, which comprises free carboxyl groups as well as crystallization inhibitors.

In known formulations utilizing (meth)acrylate based pressure sensitive adhesives, ketamine is dissolved in the (meth)acrylate based pressure sensitive adhesives. This can be problematic because the saturation concentration of ketamine in the (meth)acrylate based pressure sensitive adhesives is rather low. When ketamine is present in higher concentrations in (meth)acrylate based pressure sensitive adhesives, the ketamine is prone to recrystallization in the (meth)acrylate based pressure sensitive adhesives. Higher concentrations of ketamine in (meth)acrylate based pressure sensitive adhesives can thus usually only be achieved by additionally using crystallization inhibitors and solubility enhancers. In all cases ketamine is prone to recrystallization in (meth)acrylate based pressure sensitive adhesives which negatively affects the stability of such formulations.

Further, the TTSs for the administration of ketamine known from the prior art require optimization with regard to the flux of the active ingredient and the utilization of the active ingredient contained in the matrix layer. Further it is of advantage to provide formulations in which ketamine is present in a stable form without utilizing crystallization inhibitors and/or solubility enhancers.

Thus, it was an object of the present invention to provide a TTS for the administration of ketamine, which has an optimal, i.e. as high flux of active ingredient as possible, especially in the first 2 to 12 hours after application, and in which the ketamine contained in the matrix layer is utilized in an optimal manner. Further, the ketamine contained in the TTS shall be present under conditions, where it is chemically and physically as stable as possible. In addition, the TTS shall be simple in design and be economic in its production. In particular, ketamine shall be present in the TTS in a non-dissolved state, so that recrystallization can be prevented. Further, the TTS shall not comprise a crystallization inhibitor. The TTS according to the present invention shall however have a flux of ketamine that is comparable to the known formulations based on (meth)acrylate based pressure sensitive adhesives.

This task has surprisingly been solved by a transdermal therapeutic system according to claim 1.

Preferred embodiments are given in the dependent claims.

In the present disclosure, the expressions "comprising" or "containing" can also mean "consisting of".

The present invention concerns a transdermal therapeutic system comprising a backing layer, which is not permeable for the active ingredient, and at least one matrix layer on one side of the backing layer, wherein the matrix layer contains at least one pressure sensitive adhesive and ketamine or a pharmaceutically acceptable salt or solvate thereof, which is characterized in that the at least one pressure sensitive adhesive comprises a silicone pressure sensitive adhesive and wherein the matrix layer comprises at least one penetration enhancer which comprises methyl laurate.

Generally, the person skilled in the art knows several types of transdermal therapeutic systems. There are DIR (drug-in-reservoir)-systems, comprising a backing layer, a reservoir layer, an adhesive layer and optionally a detachable protective layer. In these systems, the pharmaceutically active ingredient is only present in the reservoir layer, but not in the adhesive layer, which contains at least one adhesive polymer.

Further, DIA (drug-in-adhesive)-systems are known, wherein a reservoir layer is omitted and the pharmaceutically active ingredient is present directly in the adhesive layer (also called matrix layer), which contains at least one adhesive polymer.

The advantages of DIA-systems over DIR-systems are among others a simpler production process and a lower risk of abuse. The lower risk of abuse is highly relevant in particular with regard to the active ingredient ketamine.

Thus, the transdermal therapeutic system according to the present invention is preferably a DIA-system. That is, the active ingredient, ketamine or a pharmaceutically acceptable salt or solvate thereof, is preferably present jointly with the at least one pressure sensitive adhesive in one and the same layer.

Such a TTS is characterized by its relatively simple design and thus by an economically advantageous production. Further, such a TTS has a higher flux of active ingredient compared to known TTSs.

Importantly, ketamine is only soluble in the silicone pressure sensitive adhesive in a sufficiently low concentration of not more than 1 wt.-%. With such a low solubility, ketamine is present in a non-dissolved state and is therefore not prone to recrystallization which does not require any further crystallization inhibitors and/or solubility enhancers.

The TTS according to the present inventions is therefore preferably a suspension TTS, which means that ketamine is present in a non-dissolved state in the silicone pressure sensitive adhesive. Ketamine is preferably present in the silicone pressure sensitive adhesive in a non-dissolved state in an amount of 99 % or more and only dissolved in the silicone pressure sensitive adhesive in an amount of 1 % or less.

The concentration, in particular the saturation concentration of ketamine in the silicone pressure sensitive adhesive may be determined by the following methods.

A polymer film, which partly contains the active ingredient as a crystalline suspension (donor layer), is brought into contact with the drug-free patch matrix (acceptor layer) via a diffusion membrane.

This "sandwich" is stored and the concentration of the active ingredient in the patch matrix is determined after certain time intervals following detachment from the membrane. If it remains constant, the saturation concentration is reached.

In this case, the donor is the silicone pressure sensitive adhesive. The membrane is a suitable membrane and the acceptor which shall be smaller than the donor is of the same material as the acceptor. A smaller area weight of the acceptor accelerates the achievement of the saturation concentration.

To measure the content and concentration of the active substance, the acceptor layer must be detached from the membrane.

Another method to determine the concentration, in particular the saturation concentration of ketamine in the silicone pressure sensitive adhesive is as follows:
Seed crystals as small as possible are brought into contact with the drug-loaded polymer film and their growth and dissolution behaviour is assessed microscopically at certain intervals.

Therefore, films with different concentrations of active ingredients are produced and examined microscopically for their spontaneous recrystallization behavior. The films can be prepared directly on a microscope slide.

Yet, another method to determine the concentration, in particular the saturation concentration of ketamine in the silicone pressure sensitive adhesive is as follows:
Masses with different concentrations of active ingredients are prepared in vials and examined macroscopically for their spontaneous recrystallization behavior.

The vials are not closed, thus the solvents can evaporate under room temperature conditions.

Although all three methods described above lead to essentially the same results, the "sandwich" method is preferred.

Further, the ketamine contained in the matrix layer can be utilized in an optimal manner.

Moreover, the TTS according to the present invention has a high skin tolerance.

The term "utilized in an optimal manner" denotes that the ketamine contained in the matrix layer diffuses from the matrix layer into the skin of the patient to the widest possible extent during the application of the TTS on the patient's skin so that after application as little "unutilized" active ingredient remains in the matrix layer as possible.

The term "backing layer, which is not permeable for the active ingredient," denotes that the backing layer is essentially, preferably completely, impermeable for the active ingredient ketamine.

Suitable materials for the backing layer comprise materials such as polyesters, e.g. polyethylene terephthalate, polybutylene terephthalate, polyethylene napthalate, polyolefines, such as polyethylene or polypropylene, ethylene-vinyl acetate, polyvinyl chloride, polyamide (Nylon) and/or polyurethane. The backing layer can also be composed of a composite material and preferably comprises an aluminum coated film and one of the above given materials.

A pressure sensitive adhesive comprises or is preferably a polymer, which acts as pressure sensitive adhesive, as defined in DIN EN 923:2016-03.

The TTS according to the invention comprises ketamine as pharmaceutically active ingredient.

Ketamine is (S)-(+)-2-(2-chlorophenyl)-2-(methylamino)cyclohexan-1-one ((S)-ketamine), (R)-(-)-2-(2-chlorophenyl)-2-(methylamino)cyclohexan-1-one ((R)-ketamine) as well as the racemate (RS)-(±)-2-(2-chlorophenyl)-2-(methylamino)cyclohexan-1-one. Comprised are also pharmaceutically acceptable salts and solvates of these compounds. Further comprised are mixtures of these compounds.

In the transdermal therapeutic system according to the present invention ketamine is preferably present as (S)-ketamine and/or a pharmaceutically acceptable salt thereof, like (S)-ketamine·HCl.

Most preferably, the pharmaceutically active ingredient in the transdermal therapeutic system according to the present invention comprises (S)-ketamine, which is present in the form of the ketamine free base.

In certain embodiments the TTS might comprise any further pharmaceutically active ingredient apart from ketamine, which can be for example one or more of the group of nonsteroidal anti-inflammatory drugs (NSAIDs, e.g. ibuprofen, ketoprofen, meloxicam, piroxicam, indomethacin), COX-2 inhibitors (e.g. celecoxib, etoricoxib), opioids (e.g. fentanyl, buprenorphine, morphine, codeine, oxycodone, hydrocodone, dihydromorphine, pethidine), MAOIs (irreversible and nonselective, e.g. phenelzine, tranylcypromine, isocarboxazid), MAOIs (reversible inhibitors of MAO-A, e.g. moclobemide), MAOIs (preferential inhibitor of MAO-B, e.g. deprenyl), tricyclic (and tetracyclic) antidepressants (e.g. clomipramine, imipramine, amitriptyline, nortriptyline, protriptyline, maprotiline, amoxapine, doxepin, desipramine, trimipramine), selective serotonin reuptake inhibitors (e.g. fluoxetine, sertraline, paroxetine, fluvoxamine, citalopram, escitalopram), selective noradrenaline reuptake inhibitors (e.g. reboxetine, atomoxetine), noradrenaline and dopamine reuptake inhibitor/releaser (e.g. bupropion), serotonin and noradrenaline reuptake inhibitors (e.g. venlafaxine, milnacipran, duloxetine), serotonin antagonists/reuptake inhibitors (e.g. nefazodone, trazodone), alpha2-adrenoceptor antagonist (e.g. mirtazapine).

Preferably, the silicone pressure sensitive adhesives are obtained following a condensation reaction between a silanol endblocked polydimethylsiloxane (PDMS) and a silicate, preferably a silicate resin. The silicone pressure sensitive adhesives are preferably further reacted with trimethylsilyl in order to reduce the silanol content of the adhesive polymer. The silicone pressure sensitive adhesives are then preferably diluted in the appropriate solvent, e.g. n-heptane, to obtain solvent-based silicone pressure sensitive adhesives.

In principle, the solvent is not limited. It is however preferred that a solvent is used, in which ketamine is not or only poorly soluble. Preferably ketamine shall only be soluble in the solvent in an amount of less than 3 wt.-%, preferably less than 2 wt.-% and most preferably less than 1 wt.-%.

Most preferably, the solvent comprises n-heptane or is n-heptane.

Preferably, the silicone pressure sensitive adhesives that can be used according to the present invention are pressure-sensitive adhesives produced on the basis of silicone polymers, such as dimethiconol/trimethylsiloxysilicate crosspolymer and/or a trimethylsilyl treated dimethiconol/trimethylsiloxysilicate crosspolymer, which preferably contain at least 30% by weight, in particular 35 to 95% by weight, particularly preferred 40 to 90% by weight or 40 to 60 % by weight or 45 to 55 % by weight of silicone polymer(s) with respect to the silicate.

In one embodiment, the silicone pressure sensitive adhesives that can be used according to the present invention are pressure sensitive adhesives produced on the basis of silicone polymers, such as dimethiconol/trimethylsiloxysilicate crosspolymers and/or a trimethylsilyl treated dimethiconol/trimethylsiloxysilicate crosspolymers, which preferably contain 40 wt.-% of silicone polymers and 60 wt.-% of silicate. Such an adhesives can be referred to as "medium tack adhesives".

In another embodiment, the silicone pressure-sensitive adhesives that can be used according to the present invention are pressure-sensitive adhesives produced on the basis of silicone polymers, such as dimethiconol/trimethylsiloxysilicate crosspolymers and/or a trimethylsilyl treated dimethiconol/trimethylsiloxysilicate crosspolymers, which preferably contains 45 wt.-% of silicone polymers, and 55 wt.-% of silicate. Such an adhesives can be referred to as "high tack adhesives".

Preferably, the TTS according to the present invention comprises a 1:1 (wt) ratio of a "medium tack adhesive" and a "high tack adhesive" as described above.

The advantage of such a mixture is that the so called "cold flow" can be minimized. The "cold flow" is understood as the leakage of adhesive out of the edges of the TTS, especially during storage.

All silicone pressure sensitive adhesives as described before contain preferably n-heptane as solvent.

The silicone pressure sensitive adhesives as described before are preferably present in an amount of 50 to 80 wt.-%, preferably about 60 to 75 wt.-% solids content in the respective solvent, preferably in the n-heptane.

Preferably, the silicone pressure sensitive adhesives have a peel adhesion of about 700 g/cm for the high tack adhesive and/or 900 g/cm for the medium tack adhesive as defined above.

Preferably, the silicone pressure sensitive adhesives have a shear value of about 14 kg/6.3 cm³ for the high tack adhesive and about 17 kg/6.3 cm³ for the medium tack adhesives as defined above.

Preferably, the silicone pressure sensitive adhesives have a viscosity at 0.01 rad/s and 30°C (mPas) of about 5x10⁸ mPas (5x10⁶ P) for the high tack adhesive and about 1×10¹⁰ mPas (1×10⁸ P) for the medium tack adhesives as defined above.

The use of said silicone pressure sensitive adhesives, preferably when they are based on n-heptane, is that ketamine is not dissolvable in these substances.

According to a preferred embodiment, the transdermal therapeutic system according to the present invention comprises as the pressure-sensitive adhesive at least one of the above-described silicone pressure-sensitive adhesives, which furthermore preferably contains a silicone polymer formed from polydimethyldiphenyl siloxane.

Silicone pressure-sensitive adhesives for use in the present invention may additionally contain silicone oils and/or other softeners (plasticisers).

Additionally, mixtures or condensates of silicone resins and polyorganosiloxanes also come into consideration. Amine-resistant silicone pressure-sensitive adhesives are furthermore preferred, which are characterized in that they do not contain any or only contain a few free silanol functions since the Si-OH groups are alkylated.

According to a preferred embodiment, a silicone pressure-sensitive adhesive is used which softens when heated and thereby achieves a viscosity that is suitable for incorporating one, two or more drugs in solid, as well as for application by means of slot extrusion or coating, and which, following cooling, is once again present in a non-flowable state.

The softening temperature of suitable silicone pressure-sensitive adhesives lies in the range of between 50 and 200° C., preferred between 75 and 170° C., and particularly preferred between 100 and 150° C.

Suitable silicone pressure-sensitive adhesives for use in the present invention are, for example, the hot melt pressure-sensitive adhesives BIO-PSA Q7-4201 and/or BIO-PSA 4301 of Dow Corning. Thereby, BIO-PSA Q7-4201 is a "medium tack adhesive" and BIO-PSA Q7-4301 is a "high tack adhesive" as defined above. Silicone pressure-sensitive adhesives and in particular silicone hot melt pressure-sensitive adhesives that are suitable for use in the present invention and such as are described above are known to the person skilled in the art and are commercially available.

Further, the transdermal therapeutic system according to the present invention is preferably characterized in that the at least one silicone pressure sensitive adhesive is present in the matrix layer in an amount of 70 to 95 wt.-%, preferably 75 to 95 wt.-%, more preferably 78 to 92 wt.-%, based on the total weight of the matrix layer.

Further, the transdermal therapeutic system according to the present invention is preferably characterized in that the matrix layer does not comprise any pressure sensitive adhesive that comprises (meth)acrylate groups. This has the advantage that ketamine is present in the TTS in an undissolved state. Thereby, recrystallization is prevented.

In particular, the matrix layer shall not comprise any pressure sensitive adhesive that comprises an acrylic copolymer selected from 2-ethylhexyl acrylic acetate, vinyl acetate, and 2-hydroxyethyl acrylate comprising free hydroxyl groups. Pressure sensitive adhesives that shall be avoided in the matrix layer are known under the trade name DURO-TAK, in particular DURO-TAK 87-4287, DURO-TAK 87-2516, DURO-TAK 2287 or DURO-TAK 2510 of Henkel, Germany.

The at least one penetration enhancer is a compound, which stabilizes the active ingredient and thus provides a relatively high and over a long term stable resorption of the active ingredient via the skin.

In another embodiment, at least one further penetration enhancer is selected from carboxylic acids, fatty acids, and/or fatty acid esters, such as levulinic acid, valeric acid, hexanoic acid, caprylic acid, nonanoic acid, decanoic acid, lauric acid, myristic acid, palmitic acid, stearic acid, arachidic acid, behenic acid, lignoceric acid, 3-methylbutanoic acid, neoheptanoic acid, neonanonic acid, isostearic acid, oleic acid, palmitoleic acid, linolenic acid, vaccenic acid, petroselinic acid, elaidic acid, arachidonic acid, gadoleic acid, erucic acid, methyl propionate, methyl valerate, diethyl sebacate, ethyl laurate, ethyl oleate, isopropyl decanoate, isopropyl myristate, isopropyl palmitate, and/or isopropyl oleate.

In another embodiment, the TTS according to the present invention does not comprise levulinic acid.

Further, compounds such as diethyltoluamide (DEET), propylene glycol monocaprylate, propylene glycol, polyethylene glycol, diisopropyl adipate, eugenol, transcutol, lauryl lactate and/or oleyl alcohol are suitable as penetration enhancer.

The transdermal therapeutic system according to the present invention is characterized in that the at least one penetration enhancer comprises methyl laurate, because methyl laurate has the advantage that ketamine shows a fast onset of transdermal flux.

The transdermal therapeutic system according to the present invention is more preferably characterized in that only one penetration enhancer, only methyl laurate, is present in the at least one matrix layer.

Further, the transdermal therapeutic system according to the present invention is preferably characterized in that the at least one penetration enhancer is present in the matrix layer in an amount of 1 to 15 wt.-%, preferably 2 to 12 wt.-%, most preferably about 10 wt.-%, based on the weight of the matrix layer.

Preferred is that the transdermal therapeutic system according to the present invention contains ketamine in the matrix layer in an amount of 1 to 25 wt.-%, preferably 5 to 15 wt.-%, based on the weight of the matrix layer.

The transdermal therapeutic system according to the present invention is further preferably characterized in that the matrix layer comprises at least one antioxidant.

The at least one antioxidant is a chemical compound, which prevents or reduces the oxidation of other substances, in particular of the active ingredient, and thus acts against aging of the therapeutical system. In particular, antioxidants are characterized by their effect as radical scavengers and by that they prevent oxidative decomposition of sensitive molecules, in particular of the active ingredient, effected by oxygen of the air. The at least one antioxidant is preferably selected from the group consisting of alpha-tocopherol, ascorbyl palmitate and/or dibutylhydroxytoluene.

Preferably, the transdermal therapeutic system according to the present invention contains the at least one antioxidant in the matrix layer in an amount of 0.001 to 5 wt.-%, preferably 0.01 to 2 wt.-%, based on the entire weight of the matrix layer.

Apart from the above-mentioned components, the matrix layer may further comprise common additives. According to their function, these can be classified as softeners/plasticizers, tackifiers, carriers and/or fillers. The relevant, physiologically uncritical, substances are known to the person skilled in the art.

Carriers and/or fillers such as silica gels, titanium dioxide and zinc oxide may be used in conjunction with the polymer in order to influence certain physical parameters, such as cohesion and bond strength, in the desired way.

Further, abuse deterrent agents can be added to the transdermal therapeutic system to prevent or at least reduce its abuse potential. Examples for substances that can be employed as abuse deterrent agents are bittering agents, gel forming agents, irritants, substances leading to acute gastrointestinal, cardiac or respiratory effects, substances leading to violent nausea or vomiting, substances leading to repugnant smells, substances inducing sleep, substances leading to deactivation or degradation of the active ingredient upon attempted extraction.

Further, the transdermal therapeutic system can also comprise an abuse deterrent feature that renders the active and/or the system ineffective when it is used in any other way than its intended use, i.e. transdermal application.

Preferably, the transdermal therapeutic system according to the present invention is further characterized in that the matrix layer has an area weight of 30 to 400 g/m², preferably of 50 to 250 g/m², most preferably of 70 to 150 g/m².

Preferably, the transdermal therapeutic system according to the present invention is further characterized in that the transdermal therapeutic system comprises a detachable protective layer on that side of the matrix layer on which the backing layer is not arranged.

The detachable protective layer, which is in contact with the matrix and which is detached prior to application, comprises for example the same materials as used for the production of the backing layer, provided that they are made detachable, e.g. by a silicone treatment. Other detachable protective layers are polytetrafluoroethylene, treated paper, cellophane, polyvinyl chloride and the like.

Further, the present invention relates to a transdermal therapeutic system as described above for use as a medicament.

The present invention also relates to a transdermal therapeutic system as described above for use in the treatment of major depressive disorder (MDD) (also known simply as depression). In particular the described transdermal therapeutic system can be used for the reduction of the suicidal risk and/or the treatment of treatment-resistant depression (TRD).

Major depressive disorder (MDD) is a mental disorder characterized by a pervasive and persistent low mood that is accompanied by low self-esteem and by a loss of interest or pleasure in normally enjoyable activities. Major depressive disorder is a disabling condition that adversely affects a person's family, work or school life, sleeping and eating habits, and general health.

Treatment-resistant depression (TRD) describes a condition that affects people with major depressive disorder (MDD) who do not respond adequately to a course of appropriate antidepressant medication within a certain time.

Further subtypes as recognized by The American Psychiatric Association's Diagnostic and Statistical Manual of Mental Disorders (DSM-5) are melancholic depression, atypical depression, catatonic depression, depression with anxious distress, depression with peri-partum onset and seasonal affective disorder.

The present invention also relates to a transdermal therapeutic system as described above for use in the treatment of pain.

Pain is a distressing feeling often caused by intense or damaging stimuli. Pain that lasts a long time is called chronic or persistent, and pain that resolves quickly is called acute.

Nociceptive pain is caused by stimulation of sensory nerve fibers that respond to stimuli approaching or exceeding harmful intensity (nociceptors), and may be classified according to the mode of noxious stimulation. The most common categories are thermal, mechanical and chemical. Some nociceptors respond to more than one of these modalities and are consequently designated polymodal.

Nociceptive pain may be also divided into "visceral", "deep somatic" and "superficial somatic" pain.

Neuropathic pain is caused by damage or disease affecting any part of the nervous system involved in bodily feelings (the somatosensory system). Neuropathic pain may be divided into peripheral, central, or mixed (peripheral and central) neuropathic pain. Peripheral neuropathic pain is often described as "burning", "tingling", "electrical", "stabbing" or "pins and needles".

Further, the transdermal therapeutic system of the present invention can be used in different administration schemes for example in consecutive, repeated or staggered administration.

In a consecutive administration the transdermal system is applied in intervals lasting at least 12 h to achieve in the blood plasma of an individual active ingredient concentrations.

The repeated administration is preferably carried out consecutively without delays, i.e., when the one or more TTSs according to the present invention are removed at the end of an application interval, the one or more TTSs according to the present invention for the following application interval are applied immediately. Preferably, the time interval at which there may be no TTSs according to the present invention at all applied to the body is no more than 10 minutes, more preferably no more than 5 minutes.

In a staggered administration the transdermal systems is applied in intervals lasting at least 4 h to achieve in the blood plasma of an individual active ingredient concentrations.

The staggered administration is preferably carried out once daily, twice weekly or once weekly, i.e., when the one or more TTSs according to the present invention are removed at the end of an application interval, the one or more TTSs according to the present invention for the following application interval are applied considering a dose free interval of at least 18 hours.

The application time, which is intended for the transdermal therapeutic system according to the present invention, preferably is at least 6 hours, more preferred at least 12 hours, and even more preferred at least 24 hours. The amount of active ingredient is preferably adapted to the desired application time.

In a preferred embodiment, all TTSs according to the present invention are administered on the same skin area of the individual over the total period, i.e., a given skin area of the individual is overlaid or plastered repeatedly with TTSs according to the present invention.

In another preferred embodiment, all TTSs according to the present invention are administered each time on different skin areas of the individual over the total period, i.e., a given skin area of the individual is not overlaid or plastered repeatedly with TTSs according to the present invention.

The present invention will be further described below using non-limiting examples.

### Examples

In the following examples, the following ingredients were used.
BIO-PSA Q7-4201 and BIO-PSA 4301: trimethylsilyl treated dimethiconol/trimethylsiloxysilicate crosspolymer.
DURO-TAK 387-4287: Pressure sensitive adhesive on the basis of an acrylate vinyl acetate copolymer comprising free hydroxyl groups, obtained without using a crosslinking agent.
DURO-TAK 387-2054: Pressure sensitive adhesive on the basis of an acrylate vinyl acetate copolymer comprising free carboxyl groups, obtained with using a crosslinking agent.
Plastoid B: Copolymer of butyl methacylate and methyl methacylate (crystallization inhibitor)
Transcutol: Diethylene glycol monoethyl ether (enhancer)
Eutanol HD: 9-Octadecen-1-ol (enhancer)
HPC/Isopropanol: Hydroxypropyl cellulose dissolved in isopropanol (gelling agent; solid content 2 wt.-%).
HPC/1,2-Propandiol: Hydroxypropyl cellulose dissolved in 1,2-propandiol (gelling agent; solid content 2 wt.-%)
Crosspovidone: Crosslinked polyvinylpolypyrrolidone (structure agent)

### Example 1:

The formulations of the S-ketamine-containing coating compositions of this example are summarized in Table 1 below.

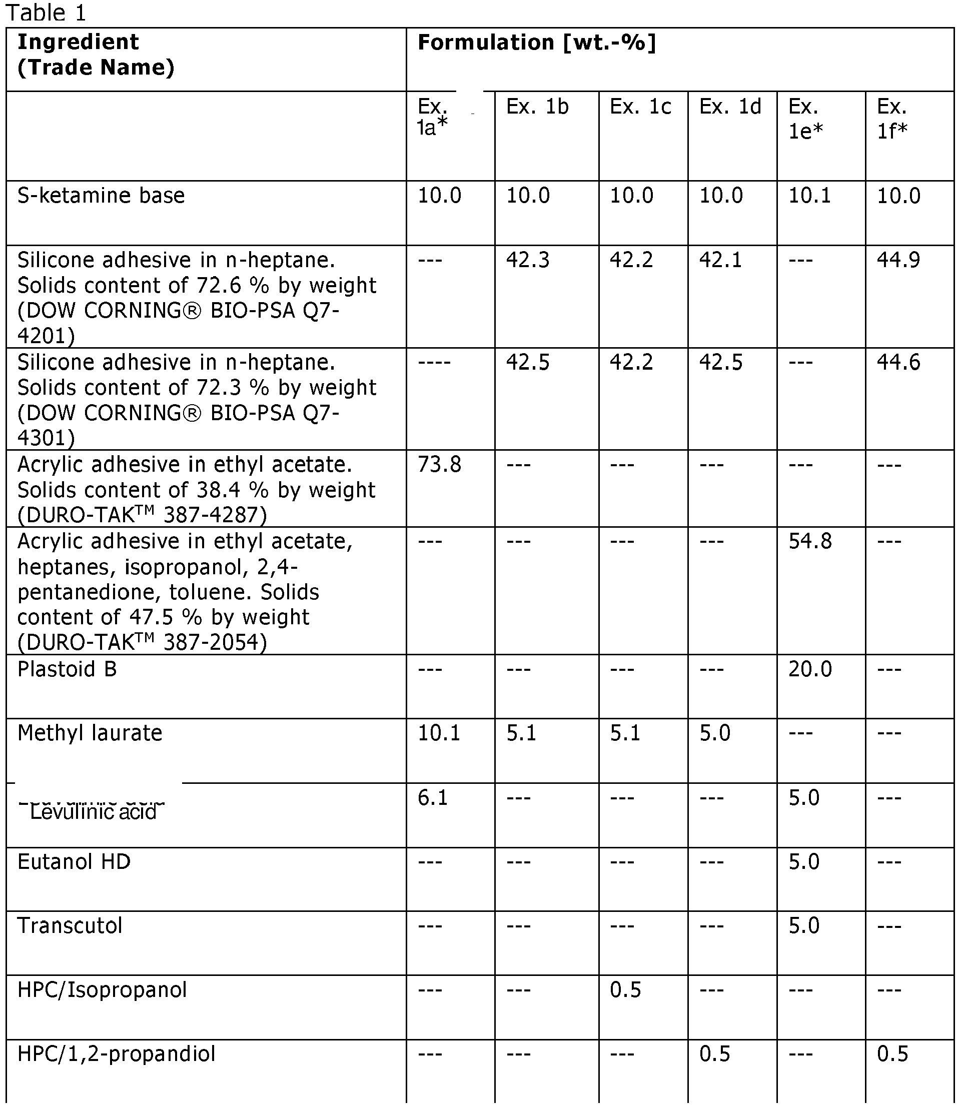

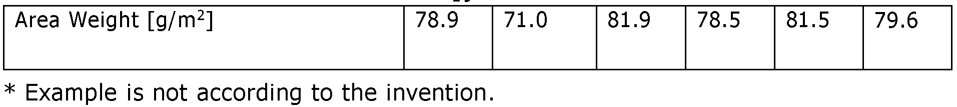

For Example 1a, a beaker was loaded with the S-ketamine base, with the solvent (ethyl acetate), the levulinic acid and the methyl laurate. The acrylic pressure sensitive adhesive polymer DURO-TAK 387-4287 was added and the mixture was then stirred at up to 300 rpm until a homogeneous mixture was obtained (stirring time is about 90 min.).

For Example 1b, a beaker was loaded with the S-ketamine base, with the solvent (n-heptane) and the methyl laurate. The mixture was stirred for about 30 min at 300 rpm and then the silicone pressure sensitive adhesives DOW CORNING BIO-PSA Q7-4201 and DOW CORNING BIO-PSA Q7-4301 were added. The mixture was then stirred at up to 900 rpm until a homogeneous mixture was obtained (stirring time is about 90 min.).

For Example 1c, a beaker was loaded with the HPC/Isopropanol solution, the methyl laurate and the S-ketamine base. The mixture was stirred for about 30 min at 300 rpm and then the silicone pressure sensitive adhesives DOW CORNING BIO-PSA Q7-4201 and DOW CORNING BIO-PSA Q7-4301 were added. The mixture was then stirred at up to 900 rpm until a homogeneous mixture was obtained (stirring time is about 90 min.).

For Example 1d, a beaker was loaded with the HPC/1,2-propandiol solution, the methyl laurate and the S-ketamine base. The mixture was stirred for about 30 min at 300 rpm and then the silicone pressure sensitive adhesives DOW CORNING BIO-PSA Q7-4201 and DOW CORNING BIO-PSA Q7-4301 were added. The mixture was then stirred at up to 900 rpm until a homogeneous mixture was obtained (stirring time is about 90 min.).

For Example 1e, a beaker was loaded with the S-ketamine base, with the solvent (ethyl acetate), the levulinic acid, the Eutanol HD and the Transcutol. The acrylic pressure sensitive adhesive polymer DURO-TAK 387-2054 was added and the mixture was then stirred at up to 300 rpm for about 30 min. The Plastoid B is added and the mixture is then stirred at up to 350 rpm until a homogeneous mixture was obtained (stirring time is about 120 min.).

For Example 1f, a beaker was loaded with the HPC/1,2-propandiol solution and the S-ketamine base. The mixture was stirred for about 30 min at 300 rpm and then the silicone pressure sensitive adhesives DOW CORNING BIO-PSA Q7-4201 and DOW CORNING BIO-PSA Q7-4301 were added. The mixture was then stirred at up to 900 rpm until a homogeneous mixture was obtained (stirring time is about 90 min.).

For Examples 1a and 1e the resulting S-ketamine-containing coating composition was coated on a polyethylene terephthalate film (siliconized, 75 µm thickness, which may function as release liner) and dried for approx. 15 min at room temperature and 15 min at 60 °C. The coating thickness gave an area weight of the matrix layer of 78.9 g/m² (Example 1a) rsp. 81.5 g/m² (Example 1e). The dried film was laminated with a polyethylene terephthalate backing layer (23 µm thickness) to provide an S-ketamine-containing self-adhesive layer structure.

For Examples 1b, 1c, 1d and 1f the resulting S-ketamine-containing coating composition was coated on a polyethylene terephthalate film (fluorpolymerized, 75 µm thickness, which may function as release liner) and dried for approx. 15 min at room temperature and 15 min at 60 °C. The coating thickness gave an area weight of the matrix layer of 71.0 g/m² (Example 1b). 81.9 g/m² (Example 1c), 78.5 g/m² (Example 1d) and 79.6 g/m² (Example 1f), respectively. The dried film was laminated with a polyethylene terephthalate backing layer (19 µm thickness) to provide an S-ketamine-containing self-adhesive layer structure.

The individual systems were then punched out from the S-ketamine-containing self-adhesive layer structure. In specific embodiments a TTS as described above can be provided with a further self-adhesive layer of larger surface area, preferably with rounded corners, comprising a pressure-sensitive adhesive matrix layer which is free of active agent. This is of advantage when the TTS, on the basis of its physical properties alone, does not adhere sufficiently to the skin and/or when the S-ketamine-containing matrix layer, for the purpose of avoiding waste, has pronounced corners (square or rectangular shapes). The systems are then punched out and sealed into pouches of the primary packaging material.

### Measurement of skin permeation rate

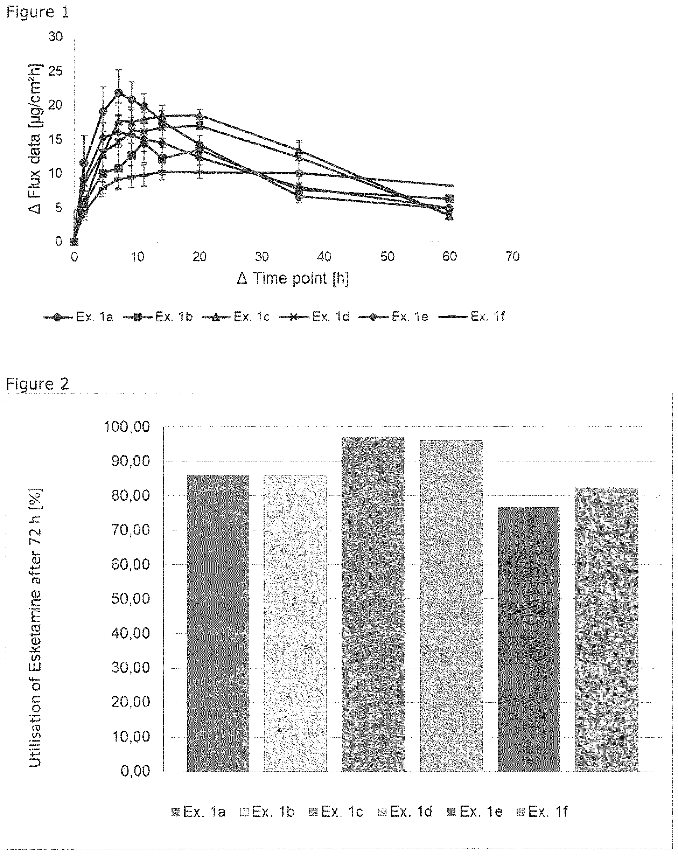

The permeated amount and the corresponding skin permeation rates of TTS prepared according to Examples 1a-1f were determined by *in vitro* experiments in accordance with the OECD Guideline (adopted April 13, 2004) and the EMA guideline on quality of transdermal patches (EMA/CHMP/QWP/608924/2014, adopted October 23, 2014), carried out with a 7.0 ml Franz diffusion cell. Split thickness human abdominal skin (female) was used. A dermatome was used to prepare skin to a thickness of 500 µm, with an intact epidermis for all TTS. Diecuts with an area of 1.152 cm² were punched from the TTS. The S-ketamine permeated amount in the receptor medium of the Franz cell (phosphate buffer solution pH 5.5 with 0.1 % saline azide as antibacteriological agent) at a temperature of 32 ± 1°C was measured after 72h and the corresponding skin permeation rate [µg/cm²*h] is calculated. The results are shown in Table 2 and Figure 1.

**Table 2**

| **Skin permeation rate with SD [µg/(cm² h)]** | | | | | | | | | | | | |
|---|---|---|---|---|---|---|---|---|---|---|---|---|
| **Elapsed time Δ[h]** | **Ex. 1a* (n** = **3)** | | **Ex. 1b (n** = **3)** | | **Ex. 1c (n** = **3)** | | **Ex. 1d (n = 3)** | | **Ex. 1e*(n = 3)** | | **Ex. 1f* (n = 3)** | |
| | **Rate** | **SD** | **Rate** | **SD** | **Rate** | **SD** | **Rate** | **SD** | **Rate** | **SD** | **Rate** | **SD** |
| **0** | 0 | 0 | 0 | 0 | 0 | 0 | 0 | 0 | 0 | 0 | 0 | 0 |
| **1.5** | 11.5 | 3.40 | 5.6 | 1.35 | 5.7 | 4.61 | 8.6 | 1.70 | 9.3 | 2.51 | 4.3 | 1.02 |
| **4.5** | 19.1 | 4.10 | 10.1 | 1.88 | 12.8 | 1.72 | 13.0 | 2.51 | 15.3 | 2.72 | 7.9 | 1.15 |
| **7** | 21.9 | 3.73 | 10.8 | 3.50 | 17.7 | 3.42 | 14.6 | 2.46 | 16.0 | 2.17 | 9.1 | 0.89 |
| **9** | 20.8 | 3.28 | 12.6 | 3.10 | 17.6 | 2.65 | 16.3 | 3.91 | 15.7 | 1.56 | 9.5 | 1.25 |
| **11** | 19.8 | 2.57 | 14.5 | 3.39 | 17.9 | 2.09 | 16.1 | 3.04 | 15.1 | 1.25 | 9.7 | 1.60 |
| **14** | 17.6 | 1.82 | 12.2 | 2.87 | 18.4 | 2.01 | 16.7 | 2.90 | 14.5 | 1.08 | 10.3 | 1.55 |
| **20** | 14.2 | 0.59 | 13.4 | 2.46 | 18.5 | 1.56 | 17.0 | 2.52 | 12.3 | 0.65 | 10.2 | 1.22 |
| **36** | 6.7 | 0.09 | 7.6 | 2.19 | 13.4 | 0.90 | 12.4 | 0.47 | 8.0 | 0.41 | 10.1 | 0.91 |
| **60** | 4.8 | 0.37 | 6.3 | 1.98 | 3.7 | 1.50 | 3.8 | 2.16 | 4.9 | 0.52 | 8.2 | 0.29 |

| | | | | | | | | | | | | |
|---|---|---|---|---|---|---|---|---|---|---|---|---|
| * Example is not according to the invention. | | | | | | | | | | | | |

### Utilization of S-ketamine

The utilization of S-ketamine at 72h was calculated based on the permeated amount of s-ketamine in the receptor and the initial S-ketamine content in the TTS. The results are shown in Table 3 and Figure 2.

**Table 3:**

| **Utilization of S-Ketamine after 72h [%]** | | | | | |
|---|---|---|---|---|---|
| Example 1a* (n = 3) | Example 1b (n = 3) | Example 1c (n = 3) | Example 1d (n = 3) | Example 1e* (n = 3) | Example 1f* (n = 3) |
| 86.0 | 86.0 | 97.1 | 96.1 | 76.7 | 82.3 |

| | | | | | |
|---|---|---|---|---|---|
| * Example is not according to the invention. | | | | | |

The results summarized in Fig. 1 to Fig. 2 show an improved utilization of the active ingredient and an improved flux of active ingredient compared to a formulation with an acrylic adhesive.

### Example 2:

The formulations of the S-ketamine-containing coating compositions of Examples 2a-d were prepared analogously as described in Example 1 and are summarized in Table 4 below.

**Table 4:**

| **Ingredient (Trade Name)** | **Formulation [wt.-%]** | | | |
|---|---|---|---|---|
| | Ex. 2a | Ex. 2b | Ex. 2c | Ex 2d |
| S-ketamine base | 10.0 | 10.0 | 10.0 | 10.0 |
| Silicone adhesive in n-heptane. Solids content of 72.6 % by weight (DOW CORNING^{®} BIO-PSA Q7-4201) | 42.2 | 39.8 | 40.0 | 42.3 |
| Silicone adhesive in n-heptane. Solids content of 72.3 % by weight (DOW CORNING^{®} BIO-PSA Q7-4301) | 42.2 | 39.8 | 39.9 | 42.1 |
| Methyl laurate | 5.1 | 10.0 | 5.1 | 5.1 |
| HPC/Isopropanol | 0.5 | 0.5 | --- | 0.5 |
| Crosspovidone | --- | --- | 5.0 | --- |
| Area Weight [g/m²] | 81.9 | 81.8 | 86.8 | 65.5 |

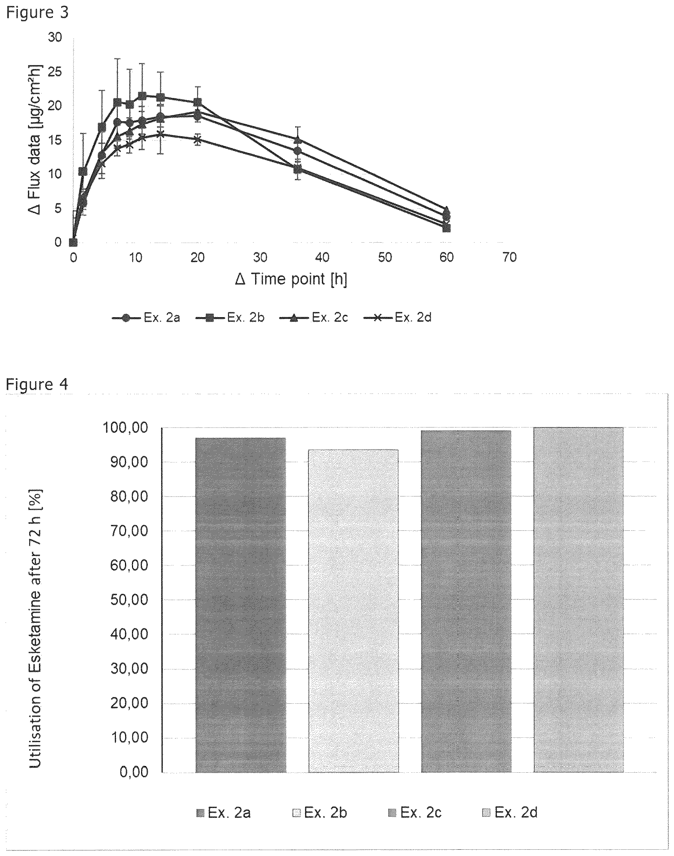

The skin permeation rate was determined analogously to Example 1 and is summarized in Figure 3.

### Utilization of S-ketamine

The utilization of S-ketamine at 72h was calculated based on the permeated amount of s-ketamine in the receptor and the initial S-ketamine content in the TTS. The results are shown in Table 5 and Figure 4.

**Table 5:**

| **Utilization of S-Ketamine after 72h [%]** | | | |
|---|---|---|---|
| Example 2a (n = 3) | Example 2b (n = 3) | Example 2c (n = 3) | Example 2d (n = 3) |
| 97.1 | 93.6 | 99.0 | 99.9 |

The results summarized in Fig. 3 to Fig. 4 show an improved utilization of the active ingredient by reducing the coating weight and a faster and higher onset of flux by using a higher concentration of enhancer. Crosspovidone is an equivalent alternative compared to HPC.

### Example 3:

The formulations of the S-ketamine-containing coating compositions of Examples 3a-h (not according to the invention) were prepared analogously as described in Example 1 and are summarized in Table 6 below.

**Table 6:**

| **Ingredient (Trade Name)** | **Formulation [wt.-%]** | | | | | | | |
|---|---|---|---|---|---|---|---|---|
| | Ex. 3a | Ex. 3b | Ex. 3c | Ex. 3d | Ex. 3e | Ex. 3f | Ex. 3g | Ex. 3h |
| S-ketamine base | 10.0 | 10.0 | 10.0 | 10.1 | 10.0 | 10.0 | 10.0 | 10.0 |
| Silicone adhesive in n-heptane. Solids content of 72.6 % by weight (DOW CORNING^{®} BIO-PSA Q7-4201) | 90.0 | --- | 44.9 | --- | --- | --- | --- | --- |
| Silicone adhesive in n-heptane. Solids content of 72.3 % by weight (DOW CORNING^{®} BIO-PSA Q7-4301) | --- | 90.0 | 45.1 | --- | --- | --- | --- | --- |
| Acrylic adhesive in ethyl acetate. Solids content of 38.4 % by weight (DURO-TAK^{™} 387-4287) | --- | --- | --- | 89.9 | --- | --- | --- | --- |
| Acrylic adhesive in ethyl acetate, heptanes, isopropanol, 2,4-pentanedion e, toluene. Solids content of 47.5 % by weight (DURO-TAK^{™} 387-2054) | --- | --- | --- | --- | 90.0 | --- | --- | --- |
| Acrylic adhesive in ethyl acetate. Solids content of 36.6 % by weight (DURO-TAK^{™} 87-9301) | --- | --- | --- | --- | --- | 90.0 | --- | --- |
| Acrylic adhesive in ethyl acetate. Solids content of 39.3 % by weight (DURO-TAK^{™} 87-4098) | --- | --- | --- | --- | --- | --- | 90.0 | --- |
| Polyisobutyl ene adhesive in n-heptane. Solids content of 36.6 % by weight (DURO-TAK^{™} 87-6908) | --- | --- | --- | --- | --- | --- | --- | 90.0 |
| Area Weight [g/m²] | 93.2 | 91.7 | 92.9 | 103.0 | 98.4 | 106.4 | 103.0 | 95.8 |

Skin permeation rate and (S)-Ketamin utilization was analyzed analogously to Example 1.

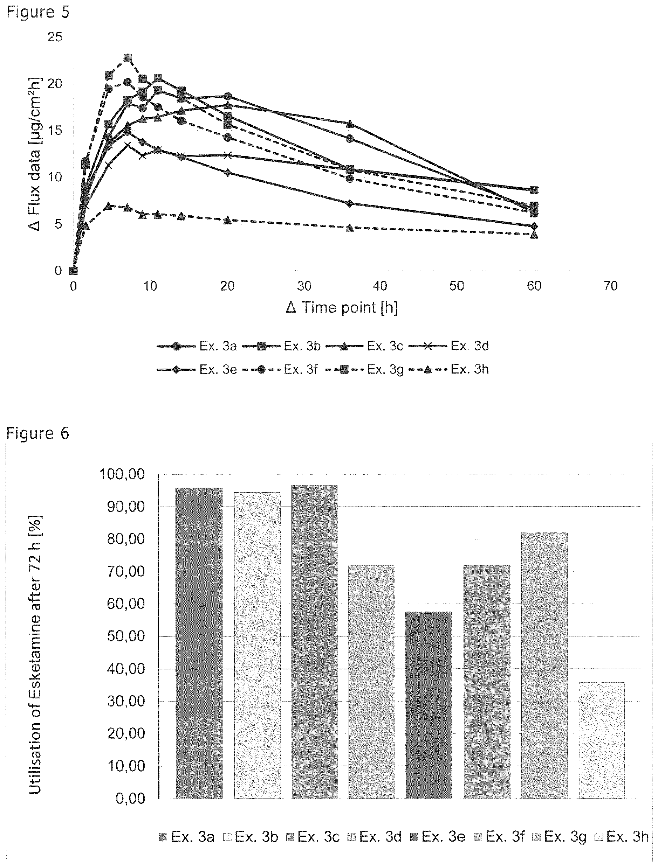

The respective results are given in Figures 5 and 6, respectively.

## Claims

1. A transdermal therapeutic system, comprising a backing layer, which is not permeable for the active ingredient, and at least one matrix layer on one side of the backing layer, wherein the matrix layer contains at least one pressure sensitive adhesive and ketamine or a pharmaceutically acceptable salt or solvate thereof, **characterized in that** the at least one pressure sensitive adhesive comprises a silicone pressure sensitive adhesive and wherein the matrix layer comprises at least one penetration enhancer which comprises methyl laurate.

2. The transdermal therapeutic system of claim 1, **characterized in that** ketamine is (S)-ketamine or a pharmaceutically acceptable salt or solvate thereof.

3. The transdermal therapeutic system of any of the preceding claims,
**characterized in that** ketamine, preferably (S)-ketamine, is present in the form of the ketamine free base.

4. The transdermal therapeutic system of any of the preceding claims,
**characterized in that** the silicone pressure sensitive adhesive comprises a dimethiconol/trimethylsiloxysilicate crosspolymer and/or a trimethylsilyl treated dimethiconol/trimethylsiloxysilicate crosspolymer.

5. The transdermal therapeutic system of any of the preceding claims,
**characterized in that** the silicone pressure sensitive adhesive is present in the matrix layer in an amount of 70 to 95 wt.-%, based on the total weight of the matrix layer.

6. The transdermal therapeutic system of any of the preceding claims,
**characterized in that** it comprises a further penetration enhancer, which is selected from levulinic acid, valeric acid, hexanoic acid, caprylic acid, nonanoic acid, decanoic acid, lauric acid, myristic acid, palmitic acid, stearic acid, arachidic acid, behenic acid, lignoceric acid, 3-methylbutanoic acid, neoheptanoic acid, neonanonic acid, isostearic acid, oleic acid, palmitoleic acid, linolenic acid, vaccenic acid, petroselinic acid, elaidic acid, arachidonic acid, gadoleic acid, erucic acid, methyl propionate, methyl valerate, diethyl sebacate, ethyl laurate, ethyl oleate, isopropyl decanoate, isopropyl myristate, isopropyl palmitate, isopropyl oleate, diethyltoluamide, propylene glycol monocaprylate, propylene glycol, polyethylene glycol, diisopropyl adipate, eugenol, diethylene glycol monoethyl ether, lauryl lactate, and/or oleyl alcohol.

7. The transdermal therapeutic system of any of the preceding claims,
**characterized in that** the matrix layer comprises the at least one penetration enhancer in an amount of 1 to 15 wt.-%, based on the total weight of the matrix layer.

8. The transdermal therapeutic system of any of the preceding claims,
**characterized in that** the matrix layer does not comprise any pressure sensitive adhesive that comprises (meth)acrylate groups.

9. The transdermal therapeutic system of any of the preceding claims,
**characterized in that** the matrix layer comprises ketamine in an amount of 1 to 25 wt.-%, based on the weight of the matrix layer.

10. The transdermal therapeutic system of any of the preceding claims,
**characterized in that** the matrix layer comprises at least one antioxidant, preferably selected from the group consisting of alpha-tocopherol, ascorbyl palmitate and/or dibutylhydroxytoluene.

11. The transdermal therapeutic system of any of the preceding claims,
**characterized in that** the matrix layer has an area weight of 30 to 400 g/m².

12. The transdermal therapeutic system of any of the preceding claims,
**characterized in that** the transdermal therapeutic system comprises a detachable protective layer on that side of the matrix layer on which the backing layer is not arranged.

13. The transdermal therapeutic system of any of the preceding claims for use as a medicament.

14. The transdermal therapeutic system of any of the preceding claims for use in the treatment of depression and/or pain.

## Patentansprüche

1. Transdermales therapeutisches System, umfassend eine Rückschicht, die nicht für den Wirkstoff durchlässig ist, und mindestens eine Matrixschicht auf einer Seite der Rückschicht, wobei die Matrixschicht mindestens einen Haftklebstoff und Ketamin oder ein pharmazeutisch verträgliches Salz oder Solvat davon enthält, **dadurch gekennzeichnet, dass** der mindestens eine Haftklebstoff einen Silikonhaftklebstoff umfasst, und wobei die Matrixschicht mindestens einen Permeationsverstärker umfasst, der Methyllaurat umfasst.

2. Transdermales therapeutisches System nach Anspruch 1,
**dadurch gekennzeichnet, dass** Ketamin (S)-Ketamin oder ein pharmazeutisch verträgliches Salz oder Solvat davon ist.

3. Transdermales therapeutisches System nach einem der vorhergehenden Ansprüche,
**dadurch gekennzeichnet, dass** Ketamin, vorzugsweise (S)-Ketamin, in Form der freien Base Ketamin vorliegt.

4. Transdermales therapeutisches System nach einem der vorhergehenden Ansprüche,
**dadurch gekennzeichnet, dass** der Silikonhaftklebstoff ein Dimethiconol/Trimethylsiloxysilikat-Kreuzpolymer und/oder ein trimethylsilylbehandeltes Dimethiconol/Trimethylsiloxysilikat-Kreuzpolymer umfasst.

5. Transdermales therapeutisches System nach einem der vorhergehenden Ansprüche,
**dadurch gekennzeichnet, dass** der Silikonhaftklebstoff in einer Menge von 70 bis 95 Gew.-%, bezogen auf das Gesamtgewicht der Matrixschicht, in der Matrixschicht vorliegt.

6. Transdermales therapeutisches System nach einem der vorhergehenden Ansprüche,
**dadurch gekennzeichnet, dass** es einen weiteren Permeationsverstärker umfasst, der ausgewählt ist aus Lävulinsäure, Valeriansäure, Hexansäure, Caprylsäure, Nonansäure, Decansäure, Laurinsäure, Myristinsäure, Palmitinsäure, Stearinsäure, Arachidinsäure, Behensäure, Lignocerinsäure, 3-Methylbutansäure, Neoheptansäure, Neonanonsäure, Isostearinsäure, Ölsäure, Palmitoleinsäure, Linolensäure, Vaccensäure, Petroselinsäure, Elaidinsäure, Arachidonsäure, Gadoleinsäure, Erucasäure, Methylpropionat, Methylvalerat, Diethylsebacat, Ethyllaurat, Ethyloleat, Isopropyldecanoat, Isopropylmyristat, Isopropylpalmitat, Isopropyloleat, Diethyltoluamid, Propylenglykolmonocaprylat, Propylenglykol, Polyethylenglykol, Diisopropyladipat, Eugenol, Diethylenglykolmonoethylether, Lauryllactat und/oder Oleylalkohol.

7. Transdermales therapeutisches System nach einem der vorhergehenden Ansprüche,
**dadurch gekennzeichnet, dass** die Matrixschicht den mindestens einen Permeationsverstärker in einer Menge von 1 bis 15 Gew.-% enthält, bezogen auf das Gesamtgewicht der Matrixschicht.

8. Transdermales therapeutisches System nach einem der vorhergehenden Ansprüche,
**dadurch gekennzeichnet, dass** die Matrixschicht keinen Haftklebstoff umfasst, der (Meth)acrylatgruppen umfasst.

9. Transdermales therapeutisches System nach einem der vorhergehenden Ansprüche,
**dadurch gekennzeichnet, dass** die Matrixschicht Ketamin in einer Menge von 1 bis 25 Gew.-%, bezogen auf das Gesamtgewicht der Matrixschicht, umfasst.

10. Transdermales therapeutisches System nach einem der vorhergehenden Ansprüche,
**dadurch gekennzeichnet, dass** die Matrixschicht mindestens ein Antioxidans umfasst, vorzugsweise ausgewählt aus der Gruppe bestehend aus alpha-Tocopherol, Ascorbylpalmitat und/oder Dibutylhydroxytoluol.

11. Transdermales therapeutisches System nach einem der vorhergehenden Ansprüche,
**dadurch gekennzeichnet, dass** die Matrixschicht ein Flächengewicht von 30 bis 400 g/m² aufweist.

12. Transdermales therapeutisches System nach einem der vorhergehenden Ansprüche,
**dadurch gekennzeichnet, dass** das transdermale therapeutische System eine ablösbare Schutzschicht auf der Seite der Matrixschicht umfasst, auf der die Rückschicht nicht angeordnet ist.

13. Transdermales therapeutisches System nach einem der vorhergehenden Ansprüche zur Verwendung als Arzneimittel.

14. Transdermales therapeutisches System nach einem der vorhergehenden Ansprüche zur Verwendung bei der Behandlung von Depressionen und/oder Schmerzen.

## Revendications

1. Système thérapeutique transdermique, comprenant une couche de support, qui n'est pas perméable au principe actif, et au moins une couche de matrice sur un côté de la couche de support, dans lequel la couche de matrice contient au moins un adhésif sensible à la pression et de la kétamine ou un sel ou un solvate pharmaceutiquement acceptable de celle-ci, **caractérisé en ce que** l'au moins un adhésif sensible à la pression comprend un adhésif sensible à la pression à base de silicone et dans lequel la couche de matrice comprend au moins un promoteur de pénétration qui comprend du laurate de méthyle.

2. Système thérapeutique transdermique selon la revendication 1, **caractérisé en ce que** la kétamine est de la (S)-kétamine ou un sel ou un solvate pharmaceutiquement acceptable de celle-ci.

3. Système thérapeutique transdermique selon l'une quelconque des revendications précédentes, **caractérisé en ce que** la kétamine, de préférence de la (S)-kétamine, est présente sous la forme de la base sans kétamine.

4. Système thérapeutique transdermique selon l'une quelconque des revendications précédentes, **caractérisé en ce que** l'adhésif sensible à la pression à base de silicone comprend un polymère croisé diméthiconol/triméthylsiloxysilicate et/ou un polymère croisé diméthiconol/triméthylsiloxysilicate traité au triméthylsilyle.

5. Système thérapeutique transdermique selon l'une quelconque des revendications précédentes, **caractérisé en ce que** l'adhésif sensible à la pression à base de silicone est présent dans la couche de matrice en une quantité de 70 à 95 % en poids, sur la base du poids total de la couche de matrice.

6. Système thérapeutique transdermique selon l'une quelconque des revendications précédentes, **caractérisé en ce qu'**il comprend un autre promoteur de pénétration, qui est sélectionné parmi de l'acide lévulinique, de l'acide valérique, de l'acide hexanoïque, de l'acide caprylique, de l'acide nonanoïque, de l'acide décanoïque, de l'acide laurique, de l'acide myristique, de l'acide palmitique, de l'acide stéarique, de l'acide arachidique, de l'acide béhénique, de l'acide lignocérique, de l'acide 3-méthylbutanoïque, de l'acide néoheptanoïque, de l'acide néonanonique, de l'acide isostéarique, de l'acide oléique, de l'acide palmitoléique, de l'acide linolénique, de l'acide vaccénique, de l'acide pétrosélinique, de l'acide élaïdique, de l'acide arachidonique, de l'acide gadoléique, de l'acide érucique, du propionate de méthyle, du valérate de méthyle, du sébacate de diéthyle, du laurate d'éthyle, de l'oléate d'éthyle, du décanoate d'isopropyle, du myristate d'isopropyle, du palmitate d'isopropyle, de l'oléate d'isopropyle, du diéthyltoluamide, du monocaprylate de propylène glycol, du propylène glycol, du polyéthylène glycol, de l'adipate de diisopropyle, de l'eugénol, de l'éther monoéthylique de diéthylène glycol, du lactate de lauryle et/ou de l'alcool oléylique.

7. Système thérapeutique transdermique selon l'une quelconque des revendications précédentes, **caractérisé en ce que** la couche de matrice comprend l'au moins un promoteur de pénétration en une quantité de 1 à 15 % en poids, sur la base du poids total de la couche de matrice.

8. Système thérapeutique transdermique selon l'une quelconque des revendications précédentes, **caractérisé en ce que** la couche de matrice ne comprend aucun adhésif sensible à la pression qui comprend des groupes (méth)acrylates.

9. Système thérapeutique transdermique selon l'une quelconque des revendications précédentes, **caractérisé en ce que** la couche de matrice comprend de la kétamine en une quantité de 1 à 25 % en poids, sur la base du poids de la couche de matrice.

10. Système thérapeutique transdermique selon l'une quelconque des revendications précédentes, **caractérisé en ce que** la couche de matrice comprend au moins un antioxydant, de préférence sélectionné dans le groupe consistant en de l'alpha-tocophérol, du palmitate d'ascorbyle et/ou du dibutylhydroxytoluène.

11. Système thérapeutique transdermique selon l'une quelconque des revendications précédentes, **caractérisé en ce que** la couche de matrice a un grammage de 30 à 400 g/m².

12. Système thérapeutique transdermique selon l'une quelconque des revendications précédentes, **caractérisé en ce que** le système thérapeutique transdermique comprend une couche protectrice détachable sur le côté de la couche de matrice sur lequel la couche de support n'est pas agencée.

13. Système thérapeutique transdermique selon l'une quelconque des revendications précédentes pour une utilisation en tant que médicament.

14. Système thérapeutique transdermique selon l'une quelconque des revendications précédentes pour une utilisation dans le traitement de la dépression et/ou de la douleur.
